# EUROPÄISCHE PATENTSCHRIFT

(11) **EP 0 781 560 B1**
(45) Veröffentlichungstag und Bekanntmachung des Hinweises auf die Patenterteilung: **04.10.2000**
(21) Anmeldenummer: 96119832.2
(22) Anmeldetag: 11.12.1996
(51) Int. Cl.: A61K 39/40, A61K 31/44

(54) **Verwendung von Immunglobulin-Präparationen zur Herstellung eines Medikaments zur oralen Verabreichung zur Behandlung und Prophylaxe chronischer Schmerzzustände**
Use of immunoglobulin preparations for the production of a drug for oral administration for treatment and prophylaxis of chronic pain conditions
Utilisation de préparations d'immunoglobulines pour la production d'un médicament apte à l'administration orale pour le traitement et prophylaxie de conditions de douleur chronique

(30) Priorität: 22.12.1995 DE 19548221
(43) Veröffentlichungstag der Anmeldung: 02.07.1997
(73) Patentinhaber: Karch, Helge, Dr., 97204 Höchberg (DE); Sprotte, Günter, Prof. Dr., 97236 Randersacker (DE)
(72) Erfinder: Sprotte, Günter, Prof. Dr., 97236 Randersacker (DE); Karch, Helge, Prof. Dr., 97204 Höchberg (DE); Lissner, Reinhard, Dr., 63937 Gönz-Weilbach (DE); Möller, Wolfgang, Dr., 61440 Oberursel (DE)
(74) Vertreter: Beil, Hans Christoph, Dr,

(56) Entgegenhaltungen:
- EP-A- 0 225 254
- EP-A- 0 338 229
- EP-A- 0 469 359
- WO-A-96/25155
- JOURNAL OF CLINICAL CHEMISTRY AND CLINICAL BIOCHEMISTRY, Bd. 28, Nr. 1, Januar 1990, BERLIN, DEUTSCHLAND, Seiten 19-23, XP000670034 W. STEPHAN ET AL.: "Antibodies from colostrum in oral immunotherapy."
- ANAESTHESIST, Bd. 41, Nr. 12, Dezember 1992, BERLIN, DEUTSCHLAND, Seiten 772-778, XP000670027 P. HÜGLER ET AL.: "Therapie der Postzosterneuralgie."
- THE AMERICAN JOURNAL OF MEDICINE, Bd. 98, Nr. 4A, 24.April 1995, NEW YORK, VSA, Seiten 44S-48S, XP000670032 A. STEERE: "Musculoskeletal manifestations of Lyme disease."
- E. Mutschler, Arzneimittelwirkungen, Lehrbuch der Pharmakologie und Toxicologie, 6. Auflage, Wissenschaftlische Verlagsgesselschaft mbH, Stuttgart.
- : "Pschyrembel Klinisches Wörterbuch, 256. Auflage", , DE GRUYTER,
- E. MUTSCHLER: "Arzneimittelwirkungen, Lehrbuch der Pharmakologie und Toxikologie, 6. Auflage", , WISSENSCHAFTLISCHE VERLAGSGESSELSCHAFT MBH, STUTTGART

## Beschreibung

Die vorliegende Erfindung betrifft die in den Ansprüchen beschriebene Verwendung von Immunglobulin-Präparationen zur Herstellung eines Medikaments zur oralen Verabreichung zur Behandlung bzw. Prophylaxe von chronischen Schmerzzuständen bei Patienten ohne pathologisch anatomische Korrelate.

Schmerzen sind ein häufiges Begleitsymptom vieler banaler aber auch ernster Erkrankungen. Die Schmerzen haben eine sinnvolle Funktion, wenn sie eine Schädigung anzeigen und als Warnsignal in der Frühphase einer Erkrankung dienen. So wird eine frühzeitige, gezielte Intervention zur Behebung der Schmerzursache ermöglicht. Oft ist die Behebung der Ursache der Schmerzen nicht mehr möglich, wie z. B. bei einer Arthrose. Ist diese Ursache bekannt, können die Schmerzen oft durch physikalische Maßnahmen oder durch analgetisch wirksamen Pharmaka gelindert werden.

Ein großes Problem stellen chronische, oft über viele Jahre und Jahrzehnte erlittene Schmerzen dar, die keiner akuten oder chronischen Grunderkrankung zugeordnet werden können und damit ohne pathologisch anatomisches Korrelat sind. Für die Patienten ergeben sich oftmals große psychische Probleme bis hin zu schweren Depressionen und Suizidversuchen, wenn die starken Schmerzen selbst mit stärksten Analgetika nur unzureichend bis gar nicht mehr beherschbar sind. Den Patienten bietet sich aus ihrer Sicht keinerlei Perspektive, da die schmerzauslösenden Faktoren vielfach unbekannt sind. Diese Patienten werden langfristig mit Opiaten oder Opioiden behandelt, wenn sie überhaupt auf diese Medikamente ansprechen. Psychopharmaka und begleitende Psychotherapie verhindern allenfalls die soziale Isolierung der Patienten. Dabei kann die Pharmakotherapie mit Betäubungsmitteln und Psychopharmaka mit erheblichen Nebenwirkungen einhergehen wie z.B. Einschränkung der Konzentrationsfähigkeit (Arbeit, Straßenverkehr, Lesen, Schreiben), Obstipation, Übelkeit etc.. Darüber hinaus ist bei der Anwendung der Opioiden die mögliche Erzeugung physischer und psychischer Abhängigkeit und anderer unerwünschter Nebenwirkungen, wie z. B. Miosis, Atemdepression, Blutdruckabfall problematisch.

In Deutschland wird mit ca. 4 000 000 Patienten mit schweren Dauerschmerzen gerechnet, davon sind mindestens 500 000 als Problemfälle anzusehen, deren Schmerzen vom niedergelassenen Arzt als therapieresistent eingestuft wurden. In anderen Ländern, wie z. B. in den USA sind die Verhältnisse bezogen auf die Gesamtbevölkerung ähnlich.

Die lang dauernden Schmerzen treten besonders häufig in Gelenken, in Muskeln und Bindegewebe (z. B. Fibromyalgien) und im Rücken auf. Diese chronischen Schmerzen verursachen z.B. in den USA einen Ausfall von mehr als 250 Mio. Arbeitstagen pro Jahr.

Von chronischen Schmerzen spricht man üblicherweise, wenn die Beschwerden länger als ein halbes Jahr bestehen. Chronische Schmerzen können im Laufe der Zeit ganz in den Vordergrund treten und ein eigenständiges Krankheitssyndrom bilden.

Die meisten klinischen Phänomene des chronischen Schmerzsyndroms werden heute mit einer Dauererregung spinaler Konvergenzneuronen erklärt. Die dauernde Erregung ist hauptsächlich durch viszerale nozizeptive Reize provozierbar, z.B. über darmständige Nozizeptoren.
Die Patienten mit chronischen Schmerzen weisen oftmals pathologisch veränderte Parameter der humoralen und zellulären Immunität auf. Häufig findet man auch antineuronale Antikörper. Ein Teil der Patienten zeigte in ersten Therapieversuchen gewisse Symptomremissionen nach Gabe von intravenösen Immunglobulin-Präparaten. Teilweise sprachen Patienten erst unter i.v.-Immunglobulin-Gabe auf Schmerzmittel, wie Opioide an. Eine dauerhafte Schmerzfreiheit durch intravenöse Immunglobulin-Gabe war jedoch nicht zu erreichen.

258 Patienten mit chronischen Schmerzsyndrom wurden mittels Immunoblot auf Antikörperaktivitäten im Serum gegen verschiedene humanpathogene Bakterien untersucht. In mehr als 80 % der Patienten waren Antikörper gegen eine oder mehrere Spezies aus der Gruppe Campylobacter jejuni, Helicobacter pylori, humanpathogenen Yersinia enterocolitica bzw. pseudotuberculosis und Borrelia burgdorferi sensu lato nachweisbar (Tabelle 1).

**Tabelle 1**

| Serologie von Patienten mit chronischen Schmerzen | | |
|---|---|---|
| Antikörper gegen | Häufigkeit bei Schmerzpatienten | Häufigkeit bei gesundem Vergleichskollektiv |
| Campylobacter jejuni | 30 % | < 4 % |
| Yersinia sp. | 20 % | < 4 % |
| Borrelia burgdorferi sensulato | 39 % | < 3 % |
| negativ | 19 % | > 90 % |

Trotz der positiven Antikörper-Befunde im Serum zeigten die Schmerzpatienten nicht häufiger die zu erwartenden Magen/Darm-Symptome als Gesunde, d.h. es bestand keine auffällige Häufung von Durchfällen oder anderen Symptomen einer akuten oder chronischen Darminfektion. Bei den meisten Patienten waren trotz des positiven Antikörpernachweises im Magen und Darm keine Bakterien der genannten Spezies nachweisbar. Selbst wenn Bakterien bei Patienten mit positivem Antikörper-Nachweis, z.B. gegen Helicobacter pylori nachweisbar waren und die Eradikation des Keimes in den meisten Fällen durch Antibiotika erfolgreich war, kam es jedoch nur in wenigen Einzelfällen zu einer Remission der Schmerzsymptome.

Wie hieraus ersichtlich ist, handelt es sich bei den Schmerzpatienten nicht um einfache Infektionen mit pathogenen Bakterien. Bei solchen akuten oder chronischen Infektionen kommt es zwar auch oft zu starken Schmerzen. Diese Schmerzen sind aber z.B. bei Magen/Darm-Infektionen lokal auf den Bauchraum lokalisiert und klar der Reizung der Schleimhäute durch z.B. Säure zuzuordnen oder auf Motilitätsstörungen und Koliken zurückzuführen.

Beim chronischen Schmerzsyndrom sind die auftretenden Schmerzen jedoch nicht lokal zuzuordnen. Selbst nach kurzzeitiger Linderung durch Antibiotika-Gabe oder intravenöser Immunglobulin-Verabreichung hielten die Schmerzen danach unver-mindert an. Oft bestand der Erfolg nur darin, daß die Patienten nach einer derartigen Therapie überhaupt auf Opioide ansprachen, um so zu einer gewissen Schmerzlinderung zu gelangen. Dabei traten jedoch die mit Opioiden verbundenen, eingangs geschilderten Nebenwirkungen auf.

Hugler et al. in Anaesthesist 41(1992), 772-78 offenbart die Therapie einer Post-Zoster-Neuralgie mit entsprechendem anti-Varizella-Zoster-Immunglobulin.

A.C. Steere in Am. J. Med. 98(4A), 1995, 44-51 betrifft das Auftreten der Fibromyalgie-Erkrankung durch das Bakterium Borrelia burgdorferi, wobei die Bakterien DNA, also eine entsprechende Infektion vorliegt.

EPA 0 469 359 betrifft die Behandlung von Magen-Darm-Erkrankungen, welche durch Campylobacter pylori verursacht werden durch entsprechende Antikörper.

W. Stephan et al., J. Chir. Chem. Chir. Biochem. 28, 1990, 19-23 beschreibt die orale Anwendung einer als Kolostrum erhaltenen Immunglobulinpräparation zur Behandlung von z.B. durch Pseudomonas aeruginosa, d.h. bakteriell bedingter Diarrhoe.

Aufgabe vorliegender Erfindung war es daher, einen Weg zu finden, Patienten mit chronischem Schmerzsyndrom eine dauerhafte Symptomfreiheit zu verschaffen bzw. Patienten vor dauerhaften Schmerzen prophylaktisch zu bewahren, wobei Nebenwirkungen bekannter, bisher eingesetzter Analgetika vermieden werden können.

Diese Aufgabe wurd erfindungsgemäß dadurch gelöst, daß man an solchen Patienten, bei denen kein pathologisch anatomisches Korrelat, ein Immunglobulin-Präparat verwendet, welches oral verabreicht wird.

Überraschenderweise wurde dabei gefunden, daß man durch die orale Gabe von Immunglobulinen bei Patienten mit chronischen Schmerzen ohne pathologische und anatomische Korrelate die Schmerzen deutlich reduzieren kann, so daß eine Behandlung mit schwersten Analgetika, wie z.B. Opioiden, nicht mehr oder nur noch in reduziertem Maße notwendig ist. Diese Wirkung war im Hinblick auf die schwache Effektivität von intravenös verabreichbarem Immunglobulin nicht zu erwarten.

Die erfindungsgemäß anzuwendenden Immunglobuline können dabei auf bekannte Weise aus Plasma, wie z.B. Humanblut, aus Eiern, aus Milch oder aus Kolostralmilch oder als monoklonale Antikörper, der Spezifität IgG, M, und/oder A, gewonnen werden. Da die Gewinnung der Immunglobuline aus Plasma relativ aufwendig und deshalb sehr teuer ist, werden die Immunglobuline, insbesondere bevorzugt aus Milch, vor allem bevorzugt aus Kolostralmilch, d.h. der Milch der ersten 5 Tage nach der Niederkunft gewonnen. Kolostralmilch hat einen hohen Gehalt an Immunglobulinen, der bis zu mehr als 50% des Gesamtproteins ausmachen kann.

Die Immunglobuline, insbesondere die der Milch oder Kolostralmilch, können vom Menschen oder von Tieren stammen. Bevorzugt wird die Milch, insbesondere Kolostralmilch von Rindern, da sie in großen Mengen anfällt und derzeit meist verworfen wird. Besonders bevorzugt werden Immunglobuline, insbesondere von Milch oder Kolostralmilch von nicht immunisierten Säugern, wie z.B. Kühen, da diese schon in hohem Maße Antikörper gegen bakterielle Antigene enthält.

Insbesondere bevorzugt sind Produkte mit Antikörperaktivität gegen humanpathogene Yersinien wie Yersinia enterocolitica oder Yersinia pseudotuberculosis, Campylobacter jejuni und Borrelia burgdorferi sensu lato.

Besonders bevorzugt weist das Präparat folgende Titer auf, bezogen auf eine Immunglobulinlösung mit 5g/100 ml Immunglobulingehalt: Im Immunoblot nach I. Autenrieth et al. (Antimicrobial Agents and Chemotherapy, 39, 1965-1969, 1995) bzw. J. Heesemann et al. (Infection and Immunity, 54, 561-567, 1986) beträgt der Antikörper gegen Campylobacter Jejuni ≥1;3200 und/oder der Antikörper-Titer gegen Yersinia enterocolitica ≥1:3200. Im Immunoblot nach H.-I Huppertz et al. (Eur.J.Pediatr. 153, 898-902, 1994) beträgt der Antikörper-Titer gegen Borrelien ≥1:200.

Die Frauen bzw. die Muttertiere können aber auch während der Schwangerschaft gegen bakterielle Erreger immunisiert werden. Für die Gewinnung von Immunglobulinen von immunisierten Spendern wird die Gewinnung der Antikörper aus Milch oder Kolostralmilch von immunisierten Kühen oder aus den Eiern von immunisierten Hühnern bevorzugt.

Für die Immunisierung werden vorzugsweise Bakterien aus der Gruppe der Campylobacter jejuni, Helicobacter pylori, humanpathogenen Yersinien und von Borrelien verwendet. Besonders bevorzugt wird die Immunisierung mit einem Gemisch aus Bakterien oder dem entsprechenden Antigengemisch von Campylobacter jejuni, Helicobacter pylori, Yersinia enterocolitica und/oder pseudotuberculosis und von Borrelia burgdorferi sensu lato. Eine Kolostralmilch immunisierter Säuger ist beispielsweise in der EP-A 0 046 909 beschrieben. Immunisierungsverfahren sind insofern bekannt.

Die Antikörper gegen Campylobactr jejuni, Helicobacter pylori, Yersinien oder Borrelien können darüberhinaus auch als monoklonale Antikörper in Zellkultur hergestellt werden, auf bekannte Weise gewonnen und dem Immunglobulin-Präparat beigefügt werden oder alleine verwendet werden.

Patienten mit chronischen Schmerzen, insbesondere mit Antikörpern gegen einen oder mehrere Bakterien aus der Gruppe Campylobacter jejuni, Helicobacter pylori, Yersinien und Borrelien, aber ohne Zeichen einer akuten oder chronischen Infektion oder Krankheit werden mit 1 bis 20 g, vorzugsweise 1-15 g und insbesondere 10 g Immunglobulin pro Tag per os behandelt.

Die Immunglobuline für die orale Therapie der chronischen Schmerzen werden nach an sich bekannten Verfahren hergestellt, wie sie z.B. in EP 0 413 187, EP 0 338 229 und EP 0 471 890 beschrieben werden. So beschreibt die EP-A 0 413 187 ein Verfahren zur Herstellung von Immunglobulin-Präparationen einer durch Fraktionierung aus Humanblut erhaltenen Proteinfraktion, die die Immunglobuline des Typs IgG, IgA und IgM in ankonzentierter Form enthält und welche - wegen der beabsichtigten intravenösen Verabreichung dort - hochrein ist.

Die EP-A 0 471 890 offenbart ein Verfahren zur Herstellung sterilfiltrierter Kolostralmilch, wobei die Kolostralmilch auch von immunisierten Säugern stammen kann.

Die EP-A 0 338 229 betrifft ein Verfahren zur Herstellung eines Präparates mit Antikörperaktivität aus Kolostralmilch nicht immunisierter Säuger. Zwar wird hier sowohl eine intravenöse als auch eine orale Verabreichbarkeit des Produktes vorgeschlagen, wobei jedoch kein Wirkungsunterschied offenbart, sondern lediglich die gute Verträglichkeit des Produktes beschrieben wird.

Ein Verfahren zur Herstellung von Immunglobulinen aus Eiern, insbesondere Hühnereiern, ist in EP 0 225 254 beschrieben. Auch hierbei können die Tiere immunisiert werden.

Die Gewinnung monoklonaler Antikörper ist ebenfalls allgemein bekannt. Ein Verfahren zur Herstellung hochreiner monoklonaler IgG-Antikörper ist beispielsweise in der EP-A 0 530 447 offenbart.

Diese Präparate enthalten Antikörperaktivitäten gegen Antigene von Campylobacter jejuni, Helicobacter pylori, humanpathogenen Yersinien sp. und/oder Borrelien. Auf welche Weise diese Antikörperaktivitäten oder eventuell andere Aktivitäten der Präparate in den Entstehungsprozeß der chronischen Schmerzen eingreifen, ist derzeit noch unbekannt. Möglicherweise liegt bei chronischen Schmerzpatienten eine gewisse Kreuzreaktivität antibakterieller Antikörper gegen neuronale Strukturen vor. Nach einer überwundenen bakteriellen Infektion könnten diese Antikörper persistieren und auf unbekanntem Wege die starken Schmerzen auslösen. Auf keinen Fall liegt jedoch eine Infektion der bekannten Art als Ursache für die Schmerzen vor. Die Wirksamkeit der erfindungsgemäßen Oraltherapie muß sich darüberhinaus von der Wirkung intravenös verabreichter Immunglobuline unterscheiden, da die oral verabreichen Antikörpermoleküle nicht in intakter, nativer Form aus dem Darm in das Blut übertreten können (wie in Fall der i.v.-Gabe), sondern im Darm entweder proteolytisch abgebaut oder ausgeschieden werden. Diese Unterschiede zeigen sich überraschenderweise in der Stärke der Wirksamkeit und der Dauerhaftigkeit.

Die bevorzugte Dosis für die orale Verabreichung der Immunglobulin-Präparate beträgt 1 bis 15 g pro Tag und ganz besonders 10 g pro Tag. Gegebenenfalls kann es von Vorteil sein, daß das Immunglobulin-Präparat kombiniert oral und inravenös verabreicht wird, wobei die orale Verabreichung insbesondere 1 bis 10 g pro Tag und ganz besonders bevorzugt 10 g pro Tag betragen und die intravenöse insbesondere 5 bis 10 g pro Tag und ganz besonders 10 g pro Tag betragen kann. Bei der intravenösen Verabreichung muß es sich dabei um humane Immunglobuline handeln.

Die Behandlungsdauer kann bei der oralen Verabreichung einige Tage bis zu einigen Wochen betragen. Die gegebenenfalls durchgeführte intravenöse Zusatztherapie erstreckt sich auf 2 bis 5 Tage in einem solchen Behandlungszeitraum.

Darüberhinaus hat es sich als vorteilhaft erwiesen, vor oder gleichzeitig mit Verabreichung des Immunglobulin-Präparates ein Mittel zur Reduktion bzw. partiellen oder vollständigen Neutralisation der Magensäure einzusetzen. Zu diesen Mitteln gehören Protonenpumpenhemmer wie z.B. Omeprazol und H2-Blocker wie z.B. Ranitidin.

Insgesamt sprechen ca. 50% der Patienten mit chronischen Schmerzen, insbesondere solche mit positivem Antikörpernachweis gegen bakterielle Pathogene auf diese Therapie an. Prinzipiell ist aber auch die orale Gabe von Immunglobulinen bei Patienten angezeigt, bei denen z.B. eine bakterielle Infektion seit längerer Zeit überstanden ist, und zunächst Symptomfreiheit vorliegt, wobei man jedoch aufgrund einer möglichen Antikörperpersistenz prophylaktisch gemäß vorliegender Erfindung eingreifen kann.

Im folgenden wird an einigen Fallbeispielen die Wirksamkeit der erfindungsgemäßen oralen Anwendung von Immunglobulinen bei schweren chronischen Schmerzen beschrieben.

### Beispiel 1

Eine 25-jährige Frau hatte extreme Kieferschmerzen und Gesichtsschwellung nach Zahnbehandlung. Anfangs linderten Antibiotika und Antiphlogistika die Schmerzen. Im folgenden wurde eine Dauerbehandlung mit Opiaten notwendig mit starker Toleranzentwicklung, zunehmender Übelkeit und massiver Obstipation.Die intravenöse Gabe von humanem Immunglobulin (IgG) führte zu nochmaliger Schmerzverstärkung und anschließender kurzfristiger Linderung. Bei der Wiederholung der intravenösen Gabe von IgG kam es immer zu mehr Schmerzverstärkung als Linderung der Schmerzen. Die orale Gabe von täglich 1,8 g polyvalenten IgG-Immunglobulin (Sandoglobulin^{(R)}) über eine Woche bei gleichzeitiger Blockade der Säuresekretion des Magens vor jeder Einahme mit Omeprazol führte ohne zwischenzeitliche Schmerzverstärkung zu einer zunehmenden Linderung bis zur Schmerzfreiheit. Bereits nach zwei Tagen konnten die Opiate ausschleichend abgesetzt werden. Die Patientin war anschließend sechs Wochen schmerzfrei. Folgende leichte Rezidive des Gesichtsschmerzes konnten mit weiteren Therapie-Serien mit oralem IgG-Immunglobulin beseitigt werden.
Die Patientin ist wieder voll berufstätig.

### Beispiel 2

Eine Patientin mit Fibromyalgie-Syndrom, das heißt Schmerzen im gesamten Bewegungsapparat, sowie stärksten einseitigen Halsschmerzen mit motorischen Schluckstörungen war seit 15 Jahren in ärztlicher Behandlung, darunter auch stationäre Aufenthalte in Psychosomatischer Klinik. Sie zeigte eine positive Borrelien- und Campylobacter jejuni- Serologie. Nach einer hochdosierten Antibiose in insgesamt vier Serien kam es nur zu einer kurzfristigen Besserung der Schmerzen. Die Patientin zeigte eine zunehmende Opiat-Resistenz und unternahm in der Folge einen Suizid-Versuch. Zuletzt wurde sie mit monatlichen Gaben von 9 Flaschen Polamidon zu 50 mg behandelt. Die Patientin erhielt für vier Wochen je 10 g pro Tag oral einer Immunglobulin-Präparation aus Kolostralmilch nicht immunisierter Säuger, hergestellt durch Entfetten der Kolostralmilch, Caseinfällung, Abtrennung des Caseins und Konzentrierung der Kolostralmolke mit anschließender Gefriertrocknung. Das Präparat war wie folgt gekennzeichnet: Im Immunoblot ließen sich bis zu einer Verdünnung von 1:6400 gegen Yersinia enterocolitica und bis zu einer Verdünung von 1:6400 ggen Campylobacter Jejuni reagierende Antikörper nachweisen. Im Immunoblot ließen sich bis zu einer Verdünnung von 1:200 gegen Borrelia burgdoferi sensu lato reagierende Antikörper nachweisen.

Die Schmerzzustände der Patientin besserten sich dramatisch. Im Verlauf von 14 Tagen konnte das Opiat ganz abgesetzt werden. Die Depressionen verschwanden ebenso wie die Schlafstörungen. Die Patientin ist wieder arbeitsfähig. Nach 6 Monaten traten wieder Symptome der Erkrankung auf, die unter erneuter Gabe des Kolostralmilch-Präparates wieder verschwanden.

### Beispiel 3

Eine 26-jährige Patientin litt seit 9 Jahren unter Schmerzen an wechselnden Lokalitäten im Achsenskelett von servikal bis lumbosakral, und hatte zuletzt stärkste Schmerzen im Nacken - Schulter - Armbereich mit Brennschmerz in beiden Händen und Unterarmen sowie beidseitiger Epicondylitis humeri radialis. Sie hatte ein starkes Krankheitsgefühl und depressive Verstimmung. Im Serum waren Antikörper gegen Campylobacter jejuni nachweisbar. Es handelte sich nur um Antikörper der IgG-Klasse. IgA-Antikörper waren nicht nachweisbar. Dies deutete auf eine abgelaufene Campylobacter jejuni-Infektion hin.
Die Patientin erhielt sechs Wochen jeweils oral 10 g des Präparates aus Beispiel 2. Nach 10 Tagen verschwand die Depression und in kurzer Folge bildeten sich dann auch alle anderen Symptome zurück. 2 Monate nach Beendigung der Behandlung traten wieder Schmerzen in den Armen auf. Es wurde erneut über 14 Tage mit dem Kolostralmilch-Präparat behandelt, wobei nach wenigen Tagen alle Symptome wieder verschwanden. Die Arbeitsfähigkeit blieb erhalten.

### Beispiel 4

Eine 20-jährige Patientin mit rezidivierenden Schmerzen in den Kniegelenken und beiden Ellbogen (Epicondylitis) litt unter starken Schmerzschüben mit gleichzeitig einhergehenden Depressionen. Serologisch waren Antikörper gegen Yersinia enterocolitica nachweisbar. Es gab keinen Hinweis für Erkrankungen des rheumatischen Formenkreises im Sinne einer Yersinien-Arthritis. Die Patientin erhielt fünf Wochen täglich 10 g des oralen Kolostralmilch-Präparates aus Beispiel 2. Die Schmerzlinderung setzte sehr schnell ein, so daß schon nach zwei Tagen die Depressionen verschwanden. Nach einer Woche hatte die Patientin nur noch geringe Schmerzen undwar wieder arbeitsfähig. Nach Absetzen des kolostralen Immunglobulin-Präparates hielt die vollständige Symptomfreiheit an.

### Beispiel 5

Eine 76-jährige Patientin litt seit 11 Jahren an einer Trigeminus-Neuralgie. Vor 6 Jahren versagte erstmals die medikementöse Therapie mit Carbamacepin. Eine Remission der Neuralgie konnte erst durch eine Behandlung mit intravenösem IgG erzielt werden. Es kam aber wieder zu einem Rezidiv. Zwischenzeitlich konnten serologisch Antikörper gegen Borrelien und Yersinia enterocolitica nachgewiesen werden. Eine Antibiotika-Behandlung und nachfolgend eine erneute Behandlung mit intravenösem IgG brachten eine Linderung der Schmerzen, so daß keine Operation erforderlich war. Dennoch hatte die Patientin tägliche Schwierigkeiten bei der Nahrungsaufnahme wegen starker Schmerzen in der Kaumuskulatur (gleichzeitiges Schmerzsymptom im Sinne eines myofascialen Gesichtsschmerzes). Schon 4 Tage nach Beginn einer oralen Therapie mit einem oralen Immunglobulin-Präparat aus Kolostralmilch wie in Beispiel 2 beschrieben trat Symptomfreiheit auf. Ein Auslaßversuch nach drei und nach fünf Wochen führten jeweils zu erneuten Schmerzen sowohl wegen der Neuralgie als auch des myofaszialen Gesichtsschmerzes. Nach insgesamt 60-tägiger oraler Immunglobulin-Therapie war schließlich auch nach dem Absetzen eine vollständige Remission eingetreten.

### Beispiel 6

Eine 46-jährige Frau litt nach der Extraktion eines Weisheitszahnes seit sechs Jahren an einem atypischen Gesichtsschmerz. Wegen vermuteter Verletzung des Unterkiefer-Hauptnervens wurde in einer erneuten Operation der verletzte Nervenabschnitt durch ein Transplantat eine Unterschenkelnervens ersetzt. Danach kam es zu einem Sudeck-Syndrom des Unterschenkels und Fußes an der Seite der Nervenentnahme. Außerdem kam es zu einer vollständigen Harninkontinenz. Seither litt die Patientin an beiden Körperregionen unter extremen Schmerzen was letztendlich zu einer Erwerbsunfähigkeit führte. Selbst die Behandlung mit Morphium in hohen Dosen zeigte keine ausreichende Wirkung. Serologisch konnten bei der Patientin Antikörper gegen Campylobacter jejuni nachgewiesen werden. Eine fünf-malige Antibiotika-Behandlung hatte nur kurzfristige und deutlich nachlassende Effekte. Eine Therapie mit intravenösem humanem Immunglobulin G führte zu einer extremen Schmerzverstärkung, die auch bei einer Wiederholung 3 Wochen anhielt. Danach kames zu einer kurzfristigen Besserung der Schmerzen für eine Woche. Die Schmerzverstäkung wurde in Kauf genommen, da unter der i.v. Immunglobulin-Gabe im gesamten Therapieintervall die Harninkontinenz beseitigt wurde.
Die orale Gabe eines Immunglobulin-Präparates wie in Beispiel 2 beschrieben führte zu einer Schmerzlinderung. Eine optimale Schmerzlinderung wurde bei dieser Patientin durch die kombinierte Gabe des oralen bovinen Immunglobulins und der intravenösen Gabe des humanen Immunglobulin-Präparates erzielt. Die orale Gabe des Immunglobulins verhinderte die zuvor beobachtete extreme Schmerzverstärkung bei der Therapie mit i.v. Immunglobulinen. Die Morphin-Behandlung konnte drastisch reduziert werden und die Patientin ist erstmals seit 6 Jahren wieder bedingt arbeitsfähig.

## Patentansprüche

1. Verwendung einer Immunglobulin-Präparation zur Herstellung eines Medikamentes für die orale Verabreichung zur Prophylaxe und Behandlung von chronischen Schmerzzuständen ohne pathologisch anatomische Korrelate.

2. Verwendung gemäß Anspruch 1, dadurch gekennzeichnet, daß Immunglobuline aus Milch eingesetzt werden.

3. Verwendung gemäß Anspruch 2, dadurch gekennzeichnet, daß Immunglobuline aus Kolostralmilch eingesetzt werden.

4. Verwendung gemäß Anspruch 1, dadurch gekennzeichnet, daß Immunglobuline aus Plasma eingesetzt werden.

5. Verwendung gemäß einem der Ansprüche 2 bis 4, dadurch gekennzeichnet, daß Immunglobuline bovinen Ursprungs eingesetzt werden.

6. Verwendung gemäß einem der Ansprüche 2 bis 4, dadurch gekennzeichnet, daß Immunglobuline humanen Ursprungs eingesetzt werden.

7. Verwendung gemäß Anspruch 1, dadurch gekennzeichnet, daß Immunglobuline aus Eiern eingesetzt werden.

8. Verwendung gemäß Anspruch 7, dadurch gekennzeichnet, daß Immunglobuline aus Eiern von Hühnern eingesetzt werden.

9. Verwendung gemäß einem der Ansprüche 1 bis 8, dadurch gekennzeichnet, daß Immunglobuline von nicht-immunisierten Spendern eingesetzt werden.

10. Verwendung gemäß einem der Ansprüche 1 bis 8, dadurch gekennzeichnet, daß Immunglobuline von immunisierten Spendern eingesetzt werden.

11. Verwendung gemäß Anspruch 1, dadurch gekennzeichnet, daß monoklonale Antikörper eingesetzt werden.

12. Verwendung gemäß einem der Ansprüche 1 bis 11, dadurch gekennzeichnet, daß Immunglobuline mit Antikörperaktivität gegen humanpathogenen Yersinien eingesetzt werden.

13. Verwendung gemäß Anspruch 12, dadurch gekennzeichnet, daß Immunglobuline mit Antikörperaktivität gegen Yersinia enterocolitica oder Yersinia pseudotuberculosis eingestzt werden.

14. Verwendung gemäß einem der Ansprüche 1 bis 11, dadurch gekennzeichnet, daß Immunglobuline mit Antikörperaktivität gegen Campylobacter jejuni eingesetzt werden.

15. Verwendung gemäß einem der Ansprüche 1 bis 11, dadurch gekennzeichnet, daß Immunglobuline mit Antikörperaktivität gegen Borrelia burgdorferi sensu lato Stämme eingesetzt werden.

16. Verwendung gemäß einem der Ansprüche 1 bis 15, dadurch gekennzeichnet, daß zusätzlich vor oder zusammen mit der Gabe der Immunglobuline Mittel zur Reduktion bzw. zur teilweisen oder vollständigen Neutralisation der Magensäure eingesetzt werden.

17. Verwendung gemäß Anspruch 16, dadurch gekennzeichnet, daß als Mittel zur Reduktion der Magensäureproduktion Omeprazol eingesetzt wird.

18. Verwendung gemäß einem der Ansprüche 1 bis 17, gekennzeichnet durch die zusätzliche Verwendung einer Immunglobulin-Präpaparation zur Herstellung eines Medikamentes für die intravenöse Verabreichung.

19. Verwendung gemäß einem der Ansprüche 1 bis 18, dadurch gekennzeichnet, daß das Immunglobulin-Präparat Immunglobuline der Klassse IgA, IgM und/oder IgG aufweist.

20. Immunglobulinpräparat, erhältlich aus Milch, Kolostralmilch oder Plasma zur oralen Gabe zur Prophylaxe und Behandlung von chronischen Schmerzzuständen ohne pathologisch anatomische Korrelate, dadurch gekennzeichnet, daß es Antikörper gegen Campylobacter jejuni und/oder Yersinia enterocolitica bzw. pseudotuberculosis und/oder Borrelia burgdorferi aufweist.

21. Immunglobulinpräparat nach Anspruch 20, dadurch gekennzeichnet, daß es aus Milch oder Kolostralmilch von Rindern erhältlich ist.

## Claims

1. Use of an immunoglobulin preparation for the production of a medicament for oral administration for the prophylaxis and treatment of chronic pain conditions without pathologically anatomical correlates.

2. Use according to claim 1, characterised in that immunoglobulins from milk are used.

3. Use according to claim 2, characterised in that immunoglobulins from colostrous milk are used.

4. Use according to claim 1, characterised in that immunoglobulins from plasma are used.

5. Use according to one of claims 2 to 4, characterised in that immunoglobulins of bovine origin are used.

6. Use according to one of claims 2 to 4, characterised in that immunoglobulins of human origin are used.

7. Use according to claim 1, characterised in that immunoglobulins from eggs are used.

8. Use according to claim 7, characterised in that immunoglobulins from hens' eggs are used.

9. Use according to one of claims 1 to 8, characterised in that immunoglobulins of unimmunised donors are used.

10. Use according to one of claims 1 to 8, characterised in that immunoglobulins of immunised donors are used.

11. Use according to claim 1, characterised in that monoclonal antibodies are used.

12. Use according to one of claims 1 to 11, characterised in that immunoglobulins having antibody activity against human pathogenic yersinia are used.

13. Use according to claim 12, characterised in that immunoglobulins having antibody activity against Yersinia enterocolitica or Yersinia pseudotuberculosis are used.

14. Use according to one of claims 1 to 11, characterised in that immunoglobulins having antibody activity against Campylobacter jejuni are used.

15. Use according to one of claims 1 to 11, characterised in that immunoglobulins having antibody activity against Borrelia burgdorferi sensu lato strains are used.

16. Use according to one of claims 1 to 15, characterised in that in addition, prior to or together with the administration of the immunoglobulins, agents for decreasing or for partially or completely neutralising the gastric acid are used.

17. Use according to claim 16, characterised in that omeprazole is used as an agent for decreasing the production of gastric acid.

18. Use according to one of claims 1 to 17, characterised by the additional use of an immunoglobulin preparation for the production of a medicament for intravenous administration.

19. Use according to one of claims 1 to 18, characterised in that the immunoglobulin preparation contains immunoglobulins of the classes IgA, IgM and/or IgG.

20. Immunoglobulin preparation obtainable from milk, colostrous milk or plasma, for oral administration for the prophylaxis and treatment of chronic pain conditions without pathologically anatomical correlates, characterised in that it contains antibodies against Campylobacter jejuni and/or Yersinia enterocolitica or Yersinia pseudotuberculosis and/or Borrelia burgdorferi.

21. Immunoglobulin preparation according to claim 20, characterised in that it is obtainable from milk or colostrous milk of cattle.

## Revendications

1. Utilisation d'une préparation d'immunoglobulines pour la préparation d'un médicament pour l'administration orale, destiné à la prophylaxie et au traitement d'états douloureux chroniques sans corrélats anatomiques du point de vue pathologique.

2. Utilisation selon la revendication 1, caractérisée en ce que l'on utilise des immunoglobulines de lait.

3. Utilisation selon la revendication 2, caractérisée en ce que l'on utilise des immunoglobulines de colostrum.

4. Utilisation selon la revendication 1, caractérisée en ce que l'on utilise des immunoglobulines du plasma.

5. Utilisation selon l'une des revendications 2 à 4, caractérisée en ce que l'on utilise des immunoglobulines d'origine bovine.

6. Utilisation selon l'une des revendications 2 à 4, caractérisée en ce que l'on utilise des immunoglobulines d'origine humaine.

7. Utilisation selon la revendication 1, caractérisée en ce que l'on utilise des immunoglobulines d'oeufs.

8. Utilisation selon la revendication 7, caractérisée en ce que l'on utilise des immunoglobulines d'oeufs de poule.

9. Utilisation selon l'une des revendications 1 à 8, caractérisée en ce que l'on utilise des immunoglobulines de donneurs non immunisés.

10. Utilisation selon l'une des revendications 1 à 8, caractérisée en ce que l'on utilise des immunoglobulines de donneurs immunisés.

11. Utilisation selon la revendication 1, caractérisée en ce que l'on utilise des anticorps monoclonaux.

12. Utilisation selon l'une des revendications 1 à 11, caractérisée en ce que l'on utilise des immunoglobulines ayant une activité d'anticorps dirigés contre des *Yersinia* pathogènes pour l'homme.

13. Utilisation selon la revendication 12, caractérisée en ce que l'on utilise des immunoglobulines ayant une activité d'anticorps dirigés contre *Yersinia enterocolitica* ou *Yersinia pseudotuberculosis.*

14. Utilisation selon l'une des revendications 1 à 11, caractérisée en ce que l'on utilise des immunoglobulines ayant une activité d'anticorps dirigés contre *Campylobacter jejuni.*

15. Utilisation selon l'une des revendications 1 à 11, caractérisée en ce que l'on utilise des immunoglobulines ayant une activité d'anticorps dirigés contre des souches de *Borrelia burgdorferi* au sens large.

16. Utilisation selon l'une des revendications 1 à 15, caractérisée en ce que l'on utilise en outre, avant l'administration des immunoglobulines ou en même temps, des agents pour la réduction ou la neutralisation partielle ou totale de l'acide gastrique.

17. Utilisation selon la revendication 16, caractérisée en ce que l'on utilise de l'oméprazole comme agent de réduction de la production d'acide gastrique.

18. Utilisation selon l'une des revendications 1 à 17, caractérisée en ce que l'on utilise en outre une préparation d'immunoglobulines pour préparer un médicament à administrer par voie intraveineuse.

19. Utilisation selon l'une des revendications 1 à 18, caractérisée en ce que la préparation d'immunoglobulines contient des immunoglobulines des classes IgA, IgM et/ou IgG.

20. Préparation d'immunoglobulines pouvant être obtenue à partir de lait, de colostrum ou de plasma, à administrer par voie orale, pour la prophylaxie et le traitement d'états douloureux chroniques sans corrélats anatomiques du point de vue pathologique, caractérisée en ce qu'elle contient des anticorps dirigés contre *Campylobacter jejuni* et/ou *Yersinia enterocolitica* ou *pseudotuberculosis* et/ou *Borrelia burgdorferi.*

21. Préparation d'immunoglobulines selon la revendication 20, caractérisée en ce qu'elle peut être obtenue à partir de lait ou de colostrum de bovins.
